# EUROPEAN PATENT APPLICATION

(11) **EP 0 607 685 A2**
(43) Date of publication of application: **27.07.1994**
(21) Application number: 93310183.4
(22) Date of filing: 16.12.1993
(51) Int. Cl.: A61B 17/36, A61B 17/22

(54) **Ultrasonic surgical instrument**

(30) Priority: 21.12.1992 GB 9226531
(71) Applicant: SPEMBLY MEDICAL LIMITED, Andover, Hampshire SP10 4DR (GB)
(72) Inventor: Varney, Kelvin John, Andover, Hampshire SP11 7JD (GB)
(74) Representative: Turner, James Arthur

(57) **Abstract**

An ultrasonic surgical instrument (10) comprises an ultrasonic transducer (20) for generating ultrasonic vibrations and an operating tip (30). The operating tip is a unitary construction having a length of at least one wavelength of the ultrasonic vibrations generated by the ultrasonic transducer.

## Description

This invention relates to ultrasonic surgical instruments.

In some surgical operations, ultrasonic surgical instruments such as ultrasonic aspirators are used for selectively dissecting, emulsifying and removing unwanted biological material (e.g. tumours) from a patient's body tissue. One known ultrasonic surgical instrument comprises a handpiece having an ultrasonically vibrating operating tip. In use, the distal end of the operating tip is applied to the biological material, with aspiration (suction) being applied via an axial bore through the operating tip. An irrigating fluid is also supplied to the vicinity of the distal end of the tip. The biological material at the distal end of the operating tip is emulsified by the ultrasonic vibrations of the tip, and is removed by the aspiration through the axial bore.

The operating tip, an ultrasonic transducer for generating the ultrasonic vibrations and an optional transmission member linking the transducer to the tip are formed separately and include complementary threaded portions so that the parts can be tightly joined together. In this way, the instrument is constructed in sections, each of which is one half of a wavelength of the ultrasonic vibrations (as measured in the medium of that section) in length. The number of half-wavelength sections of transmission member is selected to provide an appropriate "reach" of the instrument for the frequency of vibrations chosen. For example, a phaco-emulsifier (used in ophthalmic surgery) is a short instrument and may have a total length (including the transducer) of only one wavelength. By contrast, a laparoscopic instrument may require several half-wavelength sections of the transmission member.

The use of half-wavelength sections means that the joints between the separate parts occur at points of minimum stress caused by the ultrasonic vibrations. A perceived advantage of this separable construction is that the sections may be dismantled by the user for maintenance and cleaning of the instrument, and also that the individual sections are considered to be easier to manufacture.

This invention provides an ultrasonic surgical instrument comprising: an ultrasonic transducer for generating ultrasonic vibrations; and an operating tip; in which the operating tip is a unitary construction having a length of at least one wavelength of the ultrasonic vibrations generated by the ultrasonic transducer.

The invention recognises that the facility of dismantling an ultrasonic instrument is generally unnecessary (in that there is little useful maintenance which can be performed by the user) and may also lead to a reduction in the performance and cleanliness of the instrument.

For example, in the separable instrument described above (employing threaded joints between the sections), the joints themselves can reduce the transmission efficiency of ultrasonic vibrations from the transducer to the distal end of the operating tip because of unwanted acoustic reflections at the interface between two sections. Furthermore, in order to separate the sections, spanner flats or the like have to be milled into each section; these disruptions in the outline of the sections can again lead to inefficient transmission of the vibrations to the distal end of the operating tip.

Another aspect is that of the cleanliness of the instrument. As described above, it may appear that an instrument having separable sections is better able to be cleaned by the user; however, in practice the joints between the sections can act as traps for microbes or other unwanted biological debris. There is also the possibility that the user may damage the various sections, and in particular the mating surfaces, during disassembly or reassembly.

These points are all addressed by the present invention in which an operating tip having a unitary construction and a length of at least one wavelength is employed. In other words, in the present invention a single operating tip has taken the place of a half-wavelength tip and a variable number of sections of the transmission member. There is no facility for the user to dismantle the operating tip; however, because there are no joints to require cleaning, there is no longer a need for the operating tip to be dismantled. The operating tip may be constructed with a smooth outer surface (i.e. without spanner flats) and without the need for finely machined mating surfaces between the sections, both of which features can improve the efficiency of the transmission of ultrasonic vibrations to the distal end of the tip. The elimination of the need for machined joints and spanner flats can reduce the complexity of manufacture of the instrument.

In a preferred embodiment the operating tip is turned from a single elongate blank. Although various other surgical materials could be employed, it is preferred that the blank is a titanium rod.

Although the invention is advantageous when a separate ultrasonic transducer is attached to a unitary operating tip, further benefits can be obtained in an instrument in which the operating tip comprises a body of the ultrasonic transducer. In such an instrument, no potentially unclean or lossy joints are required between the transducer and the operating tip. Manufacturing costs can be reduced, in that only one part, rather than several, has to be fabricated and the need for accurately milled jointing faces between the tip and the transducer body is removed. The construction of surrounding parts of the instrument (eg a shroud or handle) may also be simplified, since these no longer have to be separable to allow separation of the tip and transducer body.

In a preferred embodiment, the operating tip comprises an aspiration aperture, disposed substantially at the distal end of the operating tip, for communicating with a vacuum source. In this embodiment, the instrument is useful in ultrasonic surgical aspiration. In an advantageously simple embodiment, the operating tip comprises a substantially axial channel, one end of the channel communicating with the aspiration aperture and the other end of the channel being connectable to the vacuum source. Preferably the channel is a gun-drilled bore through the operating tip.

Although an instrument as defined above could be used in conjunction with a central vacuum source linked by distribution conduits to each of a number of surgical operating theatres, it is preferred that the instrument is self-contained in that the instrument comprises a vacuum pump for communicating with the aspiration aperture. The vacuum pump could, for example, be housed in a control unit connected by flexible tubing to an ultrasonic handpiece.

The ultrasonic vibrations generated by half-wavelength ultrasonic transducers are generally of insufficient amplitude to cause a desirable level of emulsification of biological tissue. It is therefore preferred that the operating tip comprises at least one amplifying section to increase the amplitude of the vibrations at the distal end of the operating tip. The amplitude increases as the cross sectional area of the operating tip decreases, so in one preferred embodiment the operating tip comprises at least two adjacent parallel-sided portions of different cross-sectional areas, thereby forming a step amplifier. In another preferred configuration, the cross-sectional area of the operating tip varies along at least a portion of the length of the operating tip according to an exponential, Fourier, catenoidal or linearly tapering function, thereby forming an amplitude amplifier. A step amplifier may be used in conjunction with, forex- ample, an exponential amplifier, by employing a step transition towards the proximal end of the operating tip and an exponential decrease in cross-sectional area towards the distal end of the operating tip. Again, the use of a unitary tip and step amplifier construction means that the number of potentially unclean and lossy joints is reduced and manufacturing costs can also be reduced. The reduction in costs again arises because only one part, rather than several, has to be fabricated and the need for accurately milled jointing faces between the tip and the amplifying section is removed. The construction of surrounding parts of the instrument (eg a shroud or handle) may also be simplified, since these no longer have to be seper- able to allow separation of the tip and amplifying section.

A preferred embodiment of ultrasonic instrument comprises an irrigant outlet aperture, disposed substantially at the distal end of the operating tip, for communicating with an irrigant fluid source. In an advantageously simple configuration, the instrument comprises a shroud disposed around at least a part of the operating tip, a gap between the shroud and the operating tip providing a path for irrigant fluid flow from the irrigant fluid source to the irrigant outlet aperture. This leads to a further advantageous feature: since there is no facility for the user to dismantle the operating tip, a simpler shroud can be fixed into place during initial assembly of the unit. This can lead to a more durable construction of the shroud.

Again, although a centralised source of irrigant could be used, it is preferred that the instrument comprises an irrigant fluid pump for communicating with the irrigant outlet aperture. As in the case of the vacuum pump, the irrigant fluid pump could be housed in a separate control unit and connected by flexible tubing to an ultrasonic handpiece.

Viewed from a second aspect this invention provides a method of manufacturing an ultrasonic surgical instrument in which, in use, ultrasonic vibrations are transmitted from an ultrasonic transducer to an operating tip, the method comprising the steps of: drilling a substantially axial bore through an elongate blank; and turning the blank about the ends of the bore to machine the operating tip, the operating tip having a length of at least one wavelength of the ultrasonic vibrations. As before, the operating tip may or may not include the body of the transducer.

The invention will now be described by way of example with reference to the accompanying drawings, throughout which like parts are referred to by like references, and in which:
Figure 1 is a schematic diagram of an ultrasonic surgical instrument according to the invention;
Figure 2 is an enlarged schematic diagram of the distal end of the operating tip of the instrument of Figure 1; and
Figures 3a to 3c are schematic diagrams of stages in the manufacture of the instrument of Figure 1.

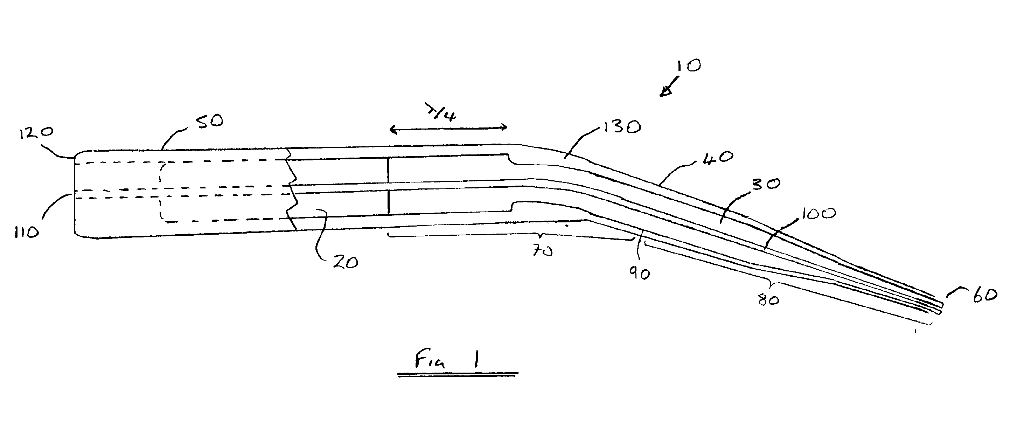

Figure 1 is a schematic diagram of an ultrasonic surgical instrument 10. An ultrasonic transducer 20 generates ultrasonic vibrations at a fixed frequency in the ultrasonic range (normally between about 20kHz and 55kHz) which vibrations are transmitted to an elongate operating tip 30 of substantially circular cross section. The transducer 20 and the operating tip 30 are housed in a plastics shroud 40, one end of which 50 forms a handle portion.

In the example shown in Figure 1, the operating tip 30 is one wavelength (k) of the ultrasonic vibrations in length. The operating tip 30 is firmly attached to the ultrasonic transducer 20, so that vibrations generated by the ultrasonic transducer 20 are transmitted to the distal end 60 of the operating tip. In use, the distal end 60 is applied to biological tissue to be emulsified.

The amplitude of the ultrasonic vibrations generated by the ultrasonic transducer 20 is small. In the absence of any measures to increase this amplitude, the vibrations of the distal end 60 of the operating tip would be corresponding small. Accordingly, two forms of amplification of the vibrations are employed, namely a step amplifier 70 and an exponential amplifier 80.

The step amplifier 70 comprises a step change in the diameter of the operating tip. The step change is located (ideally) at a node of the ultrasonic vibrations, i.e. at a quarter-wavelength from the end of the operating tip 30. The amplitude of the vibrations of the operating tip increases approximately in inverse relation to the diameter of the operating tip, so the step change in diameter causes a corresponding step increase in the vibration amplitude. In order to reduce the stress level at the step change, the internal corner is radiused.

In a similar manner to that of the step amplifier 70, the vibration amplitude is increased in the exponential amplifier 80 by reducing the diameter of the operating tip 30. However, in the case of the exponential amplifier 80, the reduction is performed gradually, according to an exponential function, over an intermediate length of the operating tip 30. This is to minimise the stress level while maintaining good amplification. Alternatively, a Fourier, catenoidal or linearly tapering function may be employed as the amplifying section.

The wavelength of the ultrasonic vibrations varies according to the diameter (or cross-sectional area) of the operating tip 30; this variation must be taken into consideration when designing an operating tip to be a particular number of half-wavelengths long.

In Figure 1, an operating tip of one wavelength in length is shown. However, in some applications, such as laparoscopic surgery, the instrument would require a longer 'reach'. In these cases, a correspondingly longer operating tip may be used. Astraightfor- ward way of extending the operating tip is simply to use a parallel-sided extension portion at a point 90. In general, in order that the distal end of the operating tip is at a point of maximum vibration, it is preferable to use an operating tip whose length is substantially equal to an integer number of half-wavelengths of the ultrasonic vibrations.

A central bore 100 through the operating tip and the ultrasonic transducer 20 links an aspiration port 110 on the handle portion 50 with an aspiration aperture at the distal end 60 of the operating tip 30. Aspiration is provided at the distal end 60 by connection of a vacuum source to the aspiration port 110. Aspiration is used to remove emulsified biological tissue from the vicinity of the distal end 60 of the operating tip 30.

The handle portion 50 also includes an irrigation port 120. Irrigant fluid, such as an isotonic saline solution, may be pumped through the irrigation port 120, through the gap 130 between the shroud 40 and the operating tip 30, to an irrigant outlet at the distal end 60 of the operating tip 30.

The vacuum source and an irrigant fluid pump may conveniently be housed in a separate control unit (not shown). Alternatively, it is common forsurgi- cal operating theatres to include "mains" supplies of vacuum and possibly irrigant from a central source.

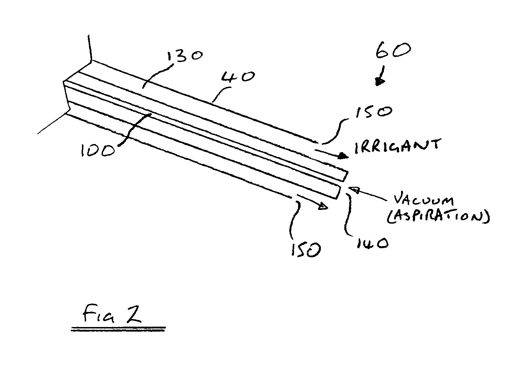

Figure 2 is an enlarged schematic diagram of the distal end 60 of the operating tip 30 shown in Figure 1. The operating tip 30 protrudes slightly from the end of the shroud 40, in order that the end of the operating tip can be applied to biological material as required. The end 140 of the axial bore 100 forms the aspiration aperture of the operating tip. As described above, irrigant fluid flows through the gap 130 between the shroud 40 and the operating tip 30, and flows out through an annular irrigant outlet 150.

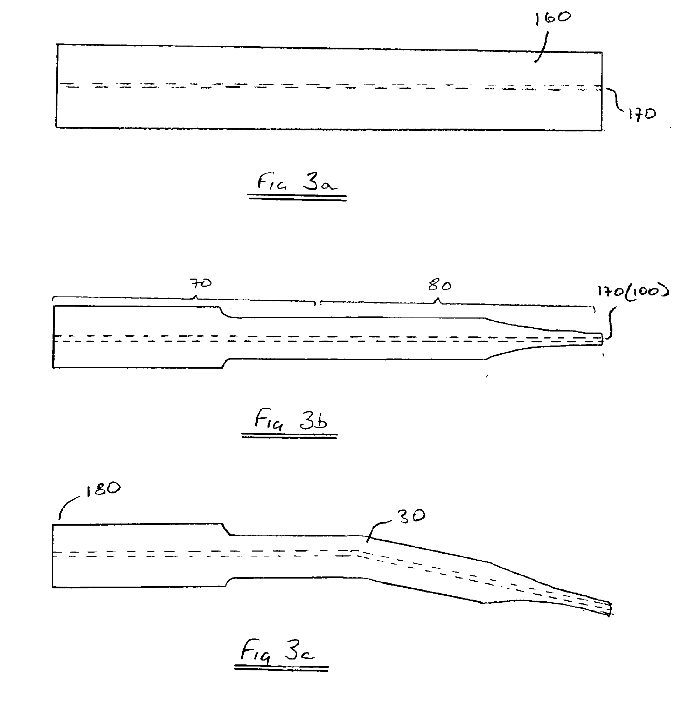

Figures 3a to 3c are schematic diagrams of three stages in the manufacture of the instrument shown in Figure 1. In Figure 3a, a titanium blank 160 of circular cross-section is gun-drilled to provide a substantially axial bore 170. In order to compensate for any deviation of the bore 170 from the axis of the blank 160, the drilled blank is mounted in a lathe for rotation about an axis defined by the two ends of the bore 170. The blank 160 is then turned to produce the overall shape of the operating tip 30, including the step amplifier 70 and the exponential amplifier80. By turning the blank around the bore 170, the bore 170 automatically lies along the axis of the turned article, thereby providing the axial bore 100 of the operating tip. Finally, as shown in Figure 3c, the operating tip 30 is bent (as necessary) to the required shape. The length of the operating tip can be varied slightly in order to tune the tip to the exact transducer frequency used.

In a modification of the above embodiment, the ultrasonic transducer 20 and the operating tip 30 may be fabricated from a single titanium blank. The ultrasonic transducer 20 comprises a number of piezoelectric ring elements stacked and clamped on a titanium body. Accordingly, as part of the manufacturing process illustrated in Figures 3 a to 3c, the body of the ultrasonic transducer 20 could be turned onto the proximal end 180 of the operating tip 30.

## Claims

1. An ultrasonic surgical instrument (10) comprising:
an ultrasonic transducer (20) for generating ultrasonic vibrations; and
an operating tip (30);
in which the operating tip is a unitary construction having a length of at least one wavelength of the ultrasonic vibrations generated by the ultrasonic transducer.

2. An instrument according to claim 1, in which the operating tip (30) is turned from a single elongate blank (160).

3. An instrument according to claim 2, in which the blank (160) is a titanium rod.

4. An instrument according to any one of the preceding claims, in which the operating tip (30) comprises a body of the ultrasonic transducer (20).

5. An instrument according to any one of the preceding claims, in which the operating tip comprises an aspiration aperture (140), disposed substantially at the distal end (60) of the operating tip (30), for communicating with a vacuum source.

6. An instrument according to claim 5, in which the operating tip (30) comprises a substantially axial channel (100), one end of the channel communicating with the aspiration aperture (140) and the other end of the channel being connectable to the vacuum source.

7. An instrument according to claim 6, in which the channel is a gun-drilled bore through the operating tip (30).

8. An instrument according to any one of claims 5 to 7, comprising a vacuum pump for communicating with the aspiration aperture (140).

9. An instrument according to any one of the preceding claims, in which the operating tip (30) comprises at least two adjacent parallel-sided portions of different cross-sectional areas, thereby forming a step amplifier.

10. An instrument according to any one of the preceding claims, in which the cross-sectional area of the operating tip (30) varies along at least a portion of the length of the operating tip according to an exponential, Fourier, catenoidal or linearly tapering function, thereby forming an amplitude amplifier.

11. An instrument according to any one of the preceding claims, comprising an irrigant outlet aperture (150), disposed substantially at the distal end of the operating tip, for communicating with an irrigant fluid source.

12. An instrument according to claim 11, comprising a shroud (40) disposed around at least a part of the operating tip, a gap between the shroud and the operating tip providing a path for irrigant fluid flow from the irrigant fluid source to the irrigant outlet aperture.

13. An instrument according to claim 11 or claim 12, comprising an irrigant fluid pump for communicating with the irrigant outlet aperture.

14. A method of manufacturing an ultrasonic surgical instrument in which, in use, ultrasonic vibrations are transmitted from an ultrasonic transducer (20) to an operating tip (30), the method comprising the steps of:
drilling a substantially axial bore (170) through an elongate blank (160); and
turning the blank about the ends of the bore to machine the operating tip, the operating tip having a length of at least one wavelength of the ultrasonic vibrations.

15. A method according to claim 14, in which the blank (160) is a titanium rod.

16. A method according to claim 14 or claim 15, in which the step of drilling a substantially axial bore (170) is performed by gun-drilling.
